# EUROPEAN PATENT SPECIFICATION

(11) **EP 3 527 556 B1**
(45) Date of publication and mention of the grant of the patent: **25.01.2023**
(21) Application number: 16919556.7
(22) Date of filing: 20.12.2016
(51) Int. Cl.: C07D 233/86, C07B 59/00, A61K 31/4166, A61P 35/00

(54) **METHOD FOR PREPARING DEUTERATED IMIDAZOLE DIKETONE COMPOUND**
VERFAHREN ZUR HERSTELLUNG EINER DEUTERIERTEN IMIDAZOL-DIKETON-VERBINDUNG
PROCÉDÉ DE PRÉPARATION D'UN COMPOSÉ D'IMIDAZOLE DICÉTONE DEUTÉRÉ

(30) Priority: 17.10.2016 CN 201610901502
(43) Date of publication of application: 21.08.2019
(73) Proprietor: Hinova Pharmaceuticals Inc., Chengdu, Sichuan 610041 (CN)
(72) Inventor: CHEN, Yuanwei, Chengdu Sichuan 610041 (CN); DU, Wu, Chengdu Sichuan 610041 (CN); KUANG, Tongtao, Chengdu Sichuan 610041 (CN); GENG, Xi, Chengdu Sichuan 610041 (CN)
(74) Representative: Altmann Stößel Dick Patentanwälte PartG mbB
(86) International application number: PCT/CN2016/110978
(87) International publication number: WO 2018/072300

(56) References cited:
- EP-A1- 2 656 841
- EP-A1- 2 792 674
- WO-A1-2011/106570
- CN-A- 101 817 787
- CN-A- 103 159 680
- CN-A- 104 803 918

## Description

### Technical Field

The present invention relates to the field of medicinal synthesis, and particularly to a method for the preparation of deuterated imidazole diketone compounds.

### Background Art

Prostatic cancer (PCa) is a common malignant tumor in male genital system, and the occurrence of this disease increases as the age, whose incidence rate has an obvious regional disparity, with Europe and America regions being higher. PCa is the second cancer causing male death, only next to lung cancer. In the past, in tumor spectrum of our country, PCa belongs to the minor disease and is not given enough attention. As the social development and progress of our country, the graying of society, the urbanization of human population, the westernization of dietary pattern, and the advancement of detection technology, the incidence rate of prostatic cancer in our country presents an obvious uptrend. The survey finished by the Second Affiliated Hospital of Tianjin Medical University and Tianjin Prostate Cancer Diagnosis and Treatment Cooperative Group in 2011 showed that the incidence rate of prostate cancer in Tianjin city was rapidly increasing, and the incidence rate of prostate cancer increased four times during the past 20 years. The patients with prostate cancer have already occupied 13.4% of urology tumor inpatients, and prostate cancer becomes a common tumor from the past rare cancer. In whole country, the incidence rate of prostate cancer presents the same trend.

Androgen is a ligand-dependent reverse transcription regulatory protein, with 1.1 × 10⁵ Dalton molecular weight. Androgen plays an very important role in the cause of prostate cancer and during its progression, as well as diseases related with male hormones such as acne, male lipsotrichia and so on.

The common treatment method for prostate cancer is surgery or androgen antagonists such as bicalutamide. However, after treatment for 2-4 years, patients can develop drug resistance, meanwhile bicalutamide further has an adverse effect of irritating cancer proliferation, and thus patients must stop using it. At present, compounds with the same bind target points as bicalutamide have already been developed, together with other drugs marketed for the treatment of metastatic prostate cancer, such as the patent CN201280052853.9.

Wherein, the following compound has improved pharmacology character:

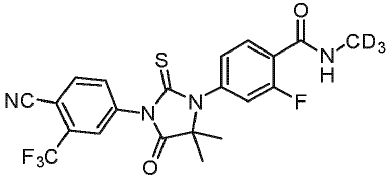

However, in the synthetic route disclosed in patent CN201280052853.9, isothiocyanate and isobutyronitrile were used for coupling. The main shortcoming in this method includes that only 11% yield of target product is obtained in the final step. That results in only 3.5% total yield of this route, beginning with the commercial available starting material. Moreover, the purification of each intermediate requires time-consuming and laborious column chromatography, and the total production time is longer, which is not desirable for industrial production.

CN 104 803 918 A discloses a preparation method of enzalutamide, comprising the following steps: adding a compound with formula F to a solvent; reacting with a methylamine source; and after the reaction, collecting the compound with formula G from the reaction products, so as to obtain the enzalutamide - wherein the reaction general formula is as follows:

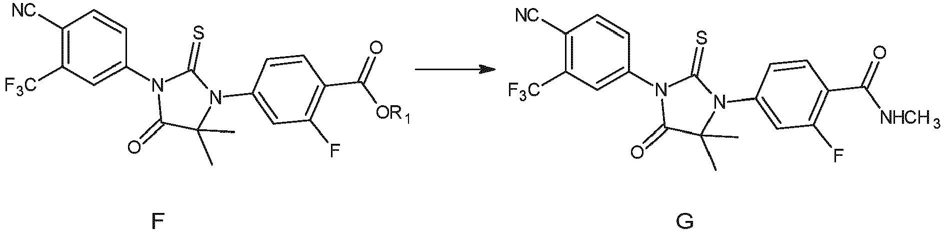

EP 2 792 674 A1 discloses imidazolidinedione compounds of formula (I) (as shown below), processes for preparation, uses and pharmaceutically compositions thereof.

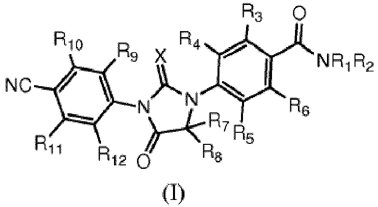

Thus, in order to improve the production efficiency and reduce the production cost, it is necessary to improve the synthetic method.

### Content of the invention

In order to solve above problems, the present invention provides a method for the preparation of deuterated imidazole diketone compounds, and it includes the following steps: 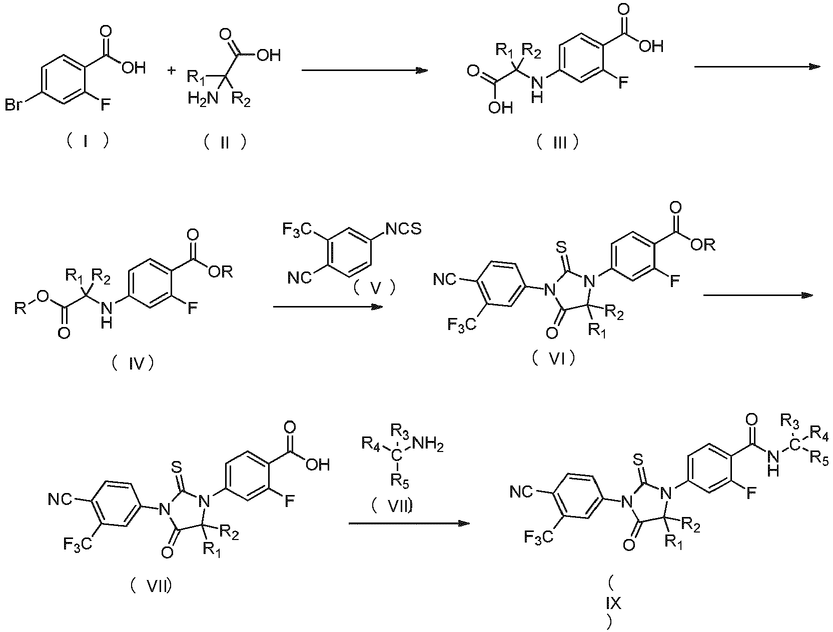
wherein R₁ and R₂ are independently selected from C₁-C₄ alkyls;
R₃, R₄, and R₅ are selected from hydrogen and deuterium, in which at least one of them is selected from deuterium;
   (1) using a compound of formula (I) and compounds of formula (II) as starting material, wherein compounds of formula (III) are obtained by a substitution reaction;
   (2) Compounds of formula (IV) are prepared by esterification of the carboxyl group in compounds of formula (III), wherein R is selected from C₁-C₆ alkyls;
   (3) Cyclization of compounds of formula (IV) and compound of formula (V) provides compounds of formula (VI);
   (4) Compounds of formula (VI) are deesterified to produce compounds of formula (VII);
   (5) compounds of formula (VII) and compounds of formula (VIII) are reacted, wherein the deuterated imidazole diketone compounds of formula (IX) are obtained by a condensation reaction forming an amide bond.

Preferably, R₁ and R₂ are both methyl.

Preferably, R₃, R₄ and R₅ are all deuterium.

Preferably, R is methyl.

Preferably, in the cyclization of step (3), solvents are the mixture of dimethyl sulfoxide and isopropyl acetate.

Preferably, the volume ratio of dimethyl sulfoxide and isopropyl acetate is 1:2.

Preferably, in step (4), said reaction is carried out in the presence of base, and the base is selected from the alkali metal hydroxides.

Preferably, said alkali hydroxides are selected from LiOH, KOH, and NaOH, and more preferably LiOH.

Preferably, in step (5), said amide condensation reaction is carried out in the presence of condensing agent, and the condensing agent is selected from isopropyl chlorocarbonate, N,N'-carbonyldiimidazole, or 2-(7-oxybenzotriazole)-N,N,N',N'-tetramethylurea hexafluorophosphate (HATU).

Preferably, in step (1), said substitution reaction is carried out in alkaline environment in the presence of Cu, CuI, and N,N-dimethylglycine.

Preferably, in step (2), said methyl esterification reagent for carboxyl group is methyl iodide. Said C₁-C₄ alkyls denote C₁, C₂, C₃, C₄ alkyls, i.e. straight chain or branch chain alkyls having 1-4 carbon atoms, such as methyl, ethyl, propyl, isopropyl, butyl, isobutyl, tert-butyl, sec-butyl, etc.

Compared with the methods in the prior art, the present invention has the following obvious advantages:
(1) The method of the present invention can realize the total yield of 40%, much better than 3.5% total yield of the current method.
(2) For the method of the present invention, the procedures are simple, not requiring column chromatography, whereby simple precipitation or crystallization means can be utilized for the purification of the products.
(3) The method of the present invention avoids the use of the extremely toxic reagent acetone cyanohydrin, and is more green and safe.

In the present invention, the abbreviations are listed in the following Table:

| | |
|---|---|
| DMSO | dimethyl sulfoxide |
| IPAc | isopropyl acetate |
| DMF | N,N-dimethylformamide |
| EA | ethyl acetate |
| THF | tetrahydrofuran |
| DMAA | N,N-dimethylglycine |
| HATU | 2-(7-oxybenzotriazole)-N,N,N',N'-tetramethylurea hexafluorophosphate |
| CDI | N,N'-carbonyldiimidazole |

By following specific examples of said embodiments, above content of the present invention is further illustrated. But it should not be construed that the scope of above subject of the present invention is limited to following examples.

### Examples

### Example 1 Preparation of 4-(1-carboxyl-1-methyl-ethylamino)-2-fluoro-benzoic acid

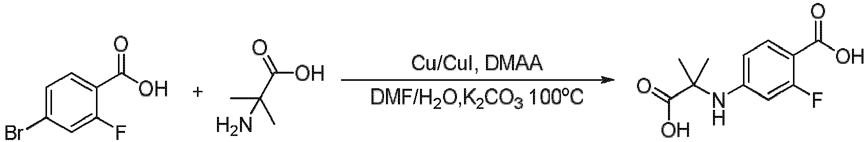

To the reaction kettle, was introduced nitrogen gas, to which were added N,N-dimethylformamide (20 L), water (2 L), 4-bromo-2-fluorobenzoic acid (2.0 kg), 2-methylalanine (2.82 kg), N,N-dimethylglycine (474 g), potassium carbonate (6.31 kg), copper powder (116 g), and CuI (348 g). Under the protection of nitrogen, the mixture was stirred at 110 °C for 16 hours. The reaction solution was cooled to the room temperature, and then ice water (30 L) was added. The pH value of solution was adjusted to 4-5 by drop adding 6N ice hydrochloric acid, and extracted with ethyl acetate twice (20L*2). The organic phase was combined, and washed with water (5 L) once. The organic phase was concentrated to dryness, to which was added dichloromethane (15 L) for pulping, then filtered, dried in vacuum at 40 °C, to provide the target compound 1.797 kg, with a yield of 81.7%.

### Example 2 Preparation of 2-fluoro-4-[(1-methoxyl-2-methyl-1-oxo-2-propyl)amino]benzoic acid methyl ester

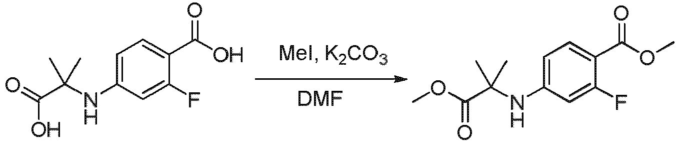

To the reaction kettle, were added N,N-dimethylformamide (7 L), 4-(1-carboxyl-1-methyl-ethylamino)-2-fluoro-benzoic acid (750 g), and potassium carbonate (6.31 kg). Then, methyl iodide (945 g) was added, and the mixture was stirred overnight. To the reaction solution, was drop added water (20 L), and stirred for crystallization, filtered, dried in vacuum at 50 °C for 16 hours, to provide the target compound 787 g, with a yield of 93.9%.
¹HNMR (DMSO, 400 MHz): 1.49 (6H, s), 3.64 (3H, s), 3.75 (3H, s), 6.16 (1H, dd, *J* = 2.1, 14.5 Hz), 6.31 (1H, dd, J = 2.1, 8.8 Hz), 7.16 (1H, s), 7.63 (1H, t, *J=* 8.8 Hz).

### Example 3 Preparation of 4-{3-[4-cyano-3-(trifluoromethyl)phenyl]-5,5-dimethyl-4-oxo-2-thio-1-imidazolidinyl}-2-fluorobenzoic acid methyl ester

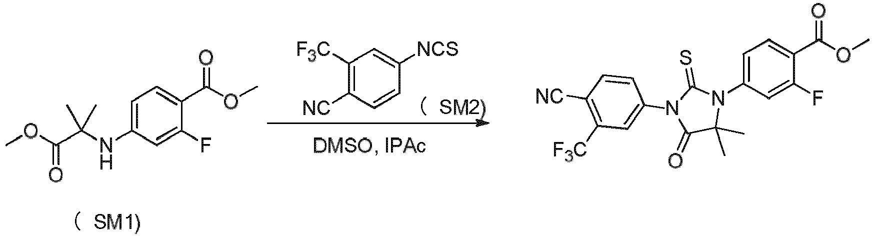

To the reaction kettle, were added 2-fluoro-4-[(1-methoxyl-2-methyl-1-oxo-2-propyl)amino]benzoic acid methyl ester (1.70 kg), 4-isothiocyanato-2-(trifluoromethyl)benzonitrile (2.88 kg), dimethyl sulfoxide (1.7 L), and iso-propyl acetate (3.4 L). Under the protection of nitrogen, the mixture was stirred at 83 °C for 40 hours. The reaction solution was concentrated under reduced pressure until no solvent was evaporated, then methanol (8.5 L) was drop added, and stirred at 0-5 °C for crystallization, filtered. The filter cake was washed with methanol and dried in vacuum at 50 °C to provide the target compound 2.3 kg, with a yield of 78.2%.
¹HNMR (DMSO, 400 MHz): 1.57 (6H, s), 3.91 (3H, s), 7.43 (1H, dd, *J =* 1.8, 8.3 Hz), 7.53 (1H, dd, *J=* 1.8, 11.2 Hz), 8.08-8.12 (2H, m), 8.30 (1H, d, J = 1.5 Hz), 8.42 (1H, d, *J=* 8.2 Hz).

According to above method, the inventors investigated the reaction solvents, and results are shown in Table 1:

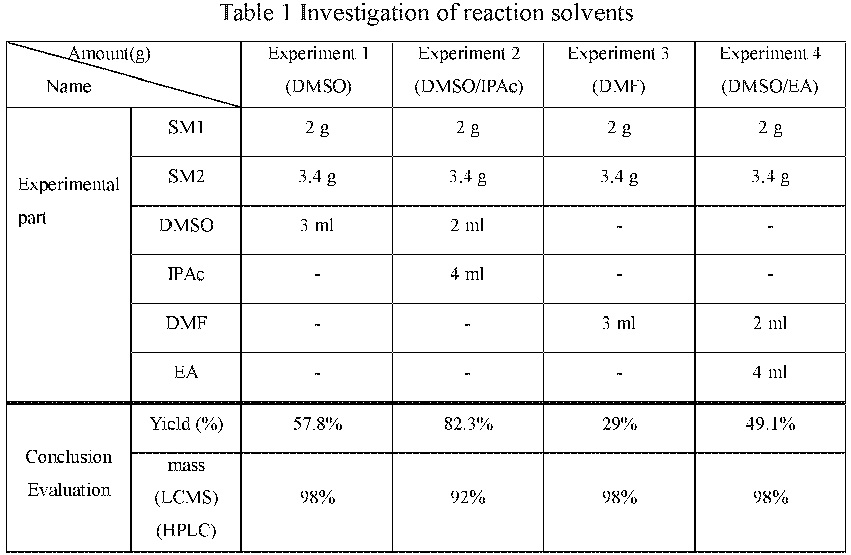

It can be shown that when the solvent is the mixture of dimethyl sulfoxide and iso-propyl acetate, the yield is higher.

### Example 4 Preparation of 4-{3-[4-cyano-3-(trifluoromethyl)phenyl]-5,5-dimethyl-4-oxo-2-thio-1-imidazolidinyl}-2-fluorobenzoic acid

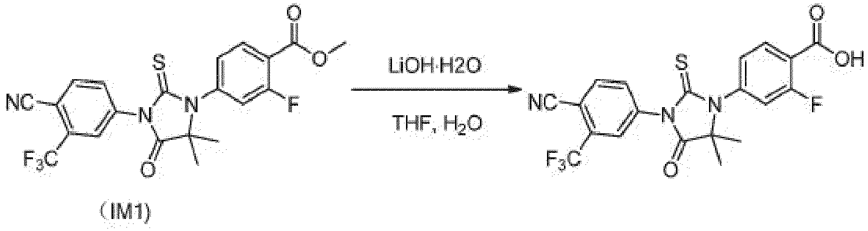

To the reaction kettle, was introduced nitrogen gas, to which were added 4-{3-[4-cyano-3-(trifluoromethyl)phenyl]-5,5-dimethyl-4-oxo-2-thio-1-imidazolidinyl}-2-fluorobenzoic acid methyl ester (1.70 kg) and tetrahydrofuran (3.4 L), and the mixture was stirred till the solution was clear. Lithium hydroxide aqueous solution (lithium hydroxide monohydrate 0.46 kg + water 3.4 L) was drop added. At the temperature of 40 °C, the mixture was incubated for 1 h. The mixture was cooled to the temperature, and water (3.4 L) was added, then the pH value was adjusted to 1-2 using brine. Ethyl acetate (13.6 L) was added for extraction. The organic phase was washed with water once, dried with anhydrous sodium sulfate, filtered, and concentrated under reduced pressure to about 3.4 L solvent remained. n-heptane (10.2 L) was added and stirred for crystallizing 1 h, then filtered and dried under vacuum to provide the target compound 1.31 kg, with a yield of 79.4%.
¹HNMR (DMSO, 400 MHz): 1.57 (6H, s), 7.39 (1H, dd, *J=* 1.6, 8.3 Hz), 7.48 (1H, dd, *J=* 1.6, 11.0 Hz), 8.06-8.12 (2H, m), 8.32 (1H, d, *J* = 1.2 Hz), 8.42 (1H, d, *J=* 8.2 Hz), 13.58 (1H, brd).

According to above method, the inventors investigated the bases, and results are shown in Table 2:

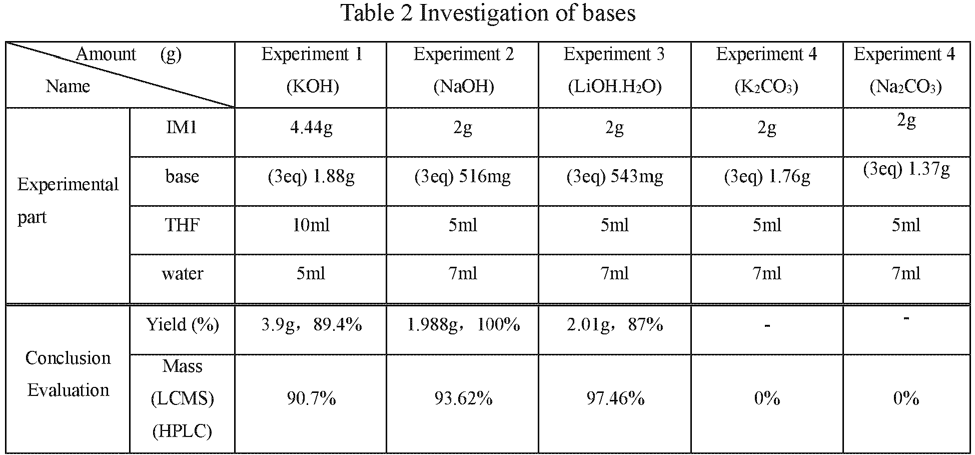

It can be found that potassium carbonate and potassium carbonate were basically not reacted, and when the base is selected from the alkali metal hydroxides, the yield is higher. Amongst, the yield is higher with LiOH, and the purity is also higher, and LiOH is preferable.

### Example 5 Preparation of 4-{3-[4-cyano-3-(trifluoromethyl)phenyl]-5,5-dimethyl-4-oxo-2-thio-1-imidazolidinyl}-2-fluoro-N-trideuteromethylbenzamide

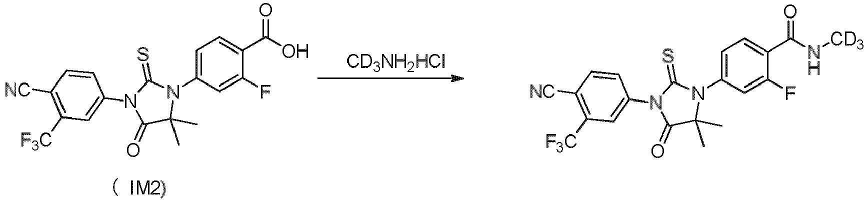

To the reaction kettle, were added 4- f 3-[4-cyano-3-(trifluoromethyl)phenyl]-5,5-dimethyl-4-oxo-2-thio-1-imidazolidinyl}-2-fluorobenzoic acid (1.30 kg) and dichloromethane (13 L), and the mixture was stirred for dissolution. N,N'-carbonyldiimidazole (0.7 kg) was added in batches, and stirred for 2h. Triethylamine (1.2 L) and deuterated methylamine hydrochlorate (302 g) were added, and stirred for 4 hours at room temperature. The reaction solution was successively washed with 1 N sodium hydroxide aqueous solution (13 L), 1 N hydrochloric acid (13 L), and water (6.5 L). The organic phase was dried with anhydrous sodium sulfate, concentrated under reduced pressure to just precipitate solids. Methyl tert-butyl ether (3.9 L) and n-heptane (3.9 L) were successively drop added, stirred, precipitated for 1 h, and filtered, to obtain crude products. The crude products were dissolved in absolute alcohol (13 L) under heating, and at the temperature of 0-5 °C, the solution was stirred and crystallized for 2 h, then filtered. At the temperature of 50 °C, the crystal was dried under vacuum for 8 hours, to provide the target compound as white solid (1.09 kg), with a yield of 80.7%. The purity was 99.8% by HPLC.
¹HNMR (DMSO, 400MHz): 1.57 (6H, s), 7.36 (1H, dd, *J* = 1.2, 8.2 Hz), 7.46 (1H, dd, *J* = 1.2, 10.7 Hz), 7.82 (1H, t, *J* = 8.2 Hz), 8.11 (1H, d, J = 8.2 Hz), 8.32 (1H, s), 8.42 (1H, d, *J* = 8.2 Hz), 8.46 (1H, s).

According to above method, the inventors investigated the condensing agents, and results are shown in Table 3:

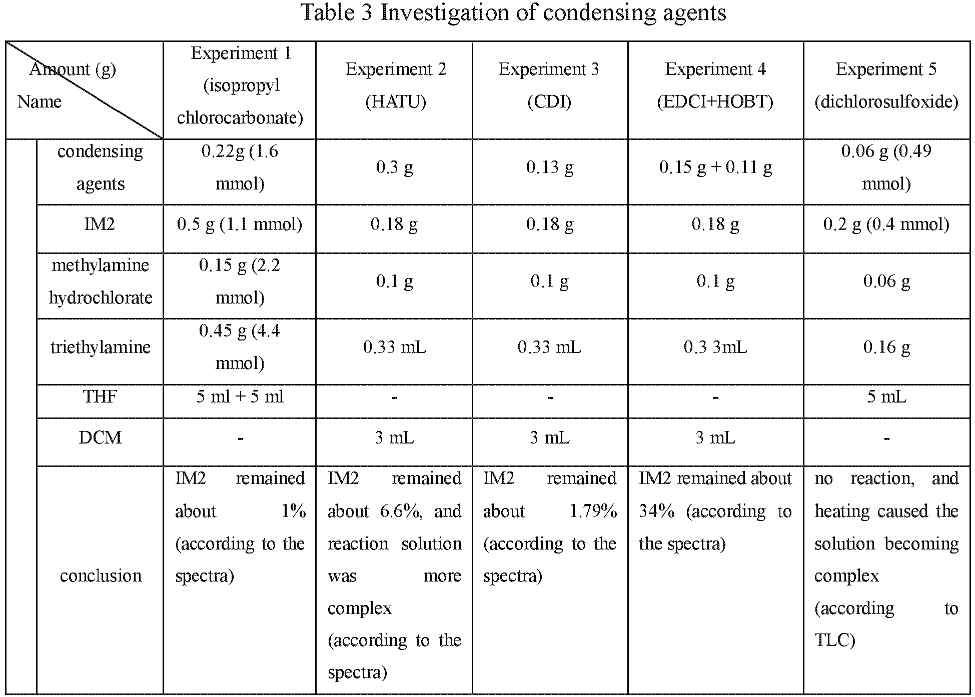

It can be shown that when isopropyl chlorocarbonate or CDI is used as the condensing agent, the yield is higher. Amongst, isopropyl chlorocarbonate need reduce the temperature to form the mixed anhydride, and thus the operation has a high standard for the equipment. Comprehensively considering, the condensing agent is preferably CDI.

### Example 6 (not forming part of the invention)

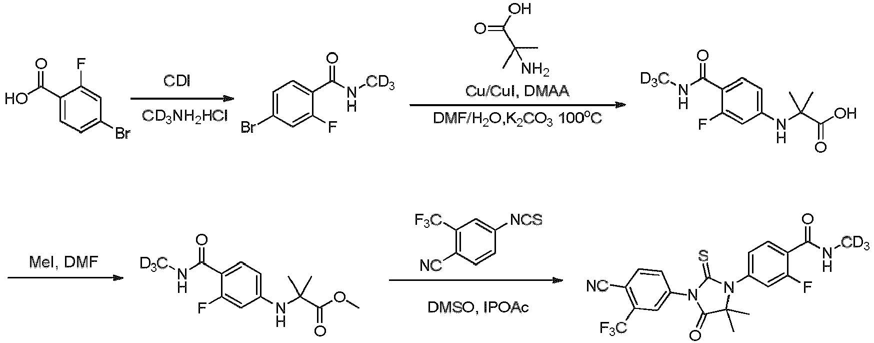

### (1) Preparation of 4-isothiocyanato-2-(trifluoromethyl)benzonitrile

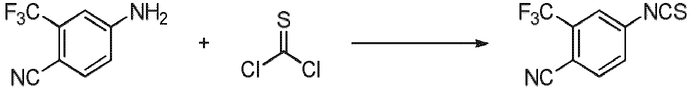

To the reaction kettle, was introduced nitrogen gas, to which were added 4-cyano-3-(trifluoromethyl)phenylamine (200 g), n-heptane (450 mL), and water (500 mL), followed by stirring to produce suspension. Thiophosgene (148 g) was drop added. The mixture was stirred at 40 °C for 16 hours, then stood for liquid separation. The water phase was extracted with n-heptane (500 mL) once, and the organic phase was combined. The solvent was removed by concentrating under reduced pressure, followed by reduced pressure distillation to provide the target compound (220 g), with a yield of 89.8%.
¹HNMR (DMSO, 400 MHz): 7.52 (1H, dd, *J=* 1.7, 8.3), 7.60 (1H, d, *J=* 1.7 Hz), 7.87 (1H, d, *J* = 8.3 Hz).

### (2) Preparation of 4-bromo-2-fluoro-N-trideuteromethylbenzamide

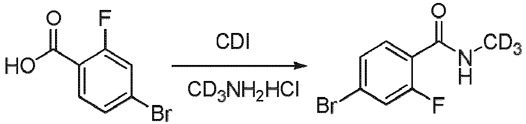

To the reaction kettle, were added 4-bromo-2-fluorobenzoic acid (50 g) and dichloromethane (500 mL), then stirred. N,N-carbonyldiimidazole (73.9 g) was added in batches, and stirred for 2 h. Triethylamine (95.5 mL) and deuterated methylamine hydrochloride (30.8 g) were added and stirred for 4 hours. The reaction solution was sequentially washed with 1 N sodium hydroxide aqueous solution (500 mL), 1 N hydrochloric acid (500 mL), and water (250 mL). The organic phase was dried with anhydrous sodium sulfate, filtered, concentrated under reduced pressure to obtain the off-white solids, that was dried 8 hours to provide the target compound as off-white solids (44.0 g), with a yield of 82.2%.

### (3) Preparation of 2-(3-fluoro-4-(trideuteromethylformamyl)phenylamino)-2-methylpropionic acid

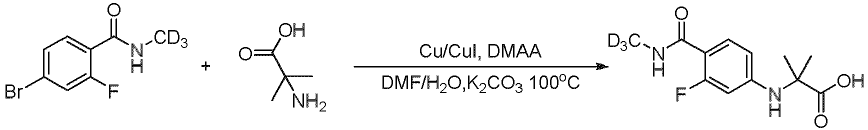

To the reaction kettle, was introduced nitrogen gas, to which were added N,N-dimethylformamide (680 mL), water (70 mL), 4-bromo-2-fluoro-N-trideuteromethylbenzamide (150 g), 2-methylalanine (199.8 g), N,N-dimethylglycine (33.3 g), potassium carbonate (446.1 g), copper powder (8.3g), and CuI (24.6 g). Under the protection of nitrogen, the mixture was stirred at 110 °C for 16 hours. The reaction solution was cooled to the room temperature, and then water (1.8 L) was added. The impurities were extracted with ethyl acetate. The pH value of solution was adjusted to 3-4 using citric acid, crystallized for 1 hour at 5 °C, filtered, and dried in vacuum to provide the target compound (100 g), with a yield of 60.9%.

### (4) 2-(3-fluoro-4-(trideuteromethylformamyl)phenylamino)-2-methylpropionic acid methyl ester

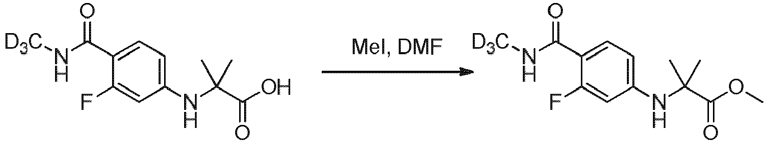

To the reaction kettle, were added N,N-dimethylformamide (630 mL), 2-(3-fluoro-4-(trideuteromethylformamyl)phenylamino)-2-methylpropionic acid (90 g), water (2.2 mL), and potassium carbonate (58.7 g). Then, methyl iodide (26.5 mL) was added, and the mixture was stirred at 40 °C for 3 hours. To the reaction solution, was added acetic acid (6.4 mL), and stirred at 60 °C for 1 hour. Water (1.35 L) was drop added, cooled to the room temperature, stirred for crystallization, filtered, dried in vacuum, to provide the target compound 91 g, with a yield of 95.2%.

### (5) 4-{3-[4-cyano-3-(trifluoromethyl)phenyl]-5,5-dimethyl-4-oxo-2-thio-1-imidazolidinyl}-2-fluoro-N-trideuteromethylbenzamide

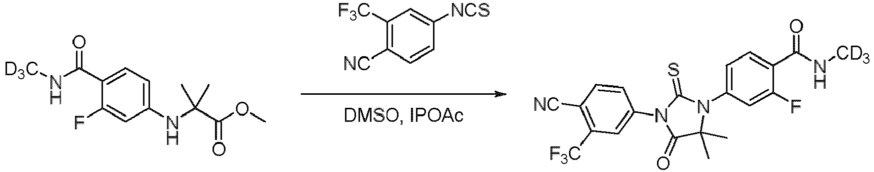

To the reaction kettle, was introduced nitrogen gas, to which were added 2-(3-fluoro-4-(trideuteromethylformamyl)phenylamino)-2-methylpropionic acid methyl ester (27.1 g), 4-isothiocyanato-2-(trifluoromethyl)benzonitrile (45.6 g), dimethyl sulfoxide (27.1 mL), and iso-propyl acetate (54.2 mL). Under the protection of nitrogen, the mixture was stirred at 83 °C for 24 hours. The reaction solution was concentrated under reduced pressure till no solvent was evaporated, then methanol (135.5 mL) was drop added, and stirred at 0-5 °C for 1 hour to crystallize, and filtered to obtain the crude product. The crude product was dissolved in absolute alcohol (250 mL) under heating and crystallized at 0-5 °C for 1 hour, filtered, and the filter cake was dried in vacuum at 50 °C to provide the target compound 34.5 g, with a yield of 73.9%.

In summary, compared with the methods in the prior art, the method according to the present invention is safer, consuming less solvents, minimizing the waste and the effect on the environment, shortening the production cycle, and improving the throughput and the total yield of the method, with a wide market outlook.

## Claims

1. A method for the preparation of deuterated imidazole diketone compounds, **characterized in that** it includes the following steps: 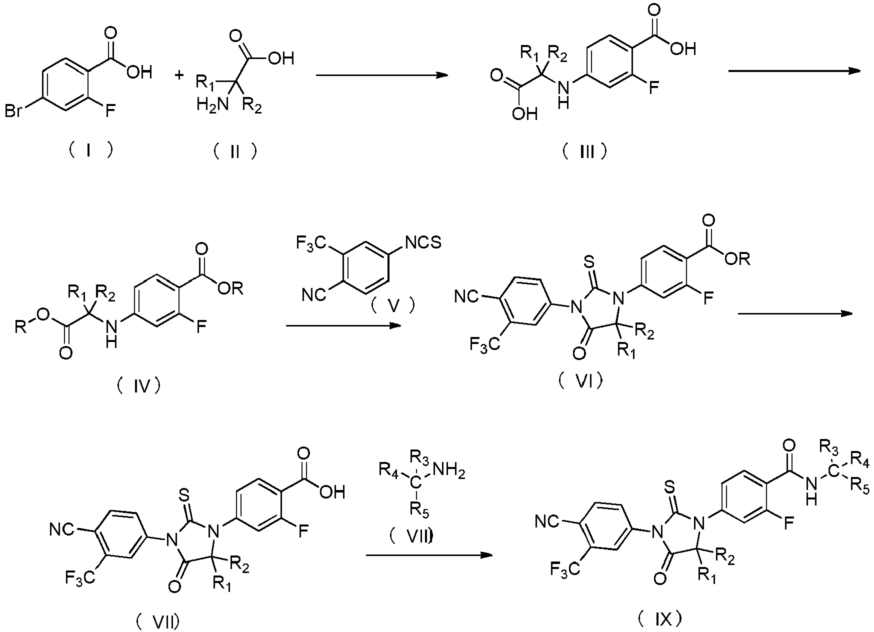
wherein R₁ and R₂ are independently selected from C₁-C₄ alkyls;
R₃, R₄, and R₅ are selected from hydrogen and deuterium, in which at least one of them is deuterium;
(1) using a compound of formula (I) and compounds of formula (II) as starting material, wherein compounds of formula (III) are obtained by a substitution reaction;
(2) Compounds of formula (IV) are prepared by esterification of the carboxyl group in compounds of formula (III), wherein R is selected from C₁-C₆ alkyls;
(3) Cyclization of compounds of formula (IV) and compound of formula (V) provides compounds of formula (VI);
(4) Compounds of formula (VI) are deesterified to produce compounds of formula (VII);
(5) compounds of formula (VII) and compounds of formula (VIII) are reacted, wherein the deuterated imidazole diketone compounds of formula (IX) are obtained by a condensation reaction forming an amide bond.

2. The method according to claim 1, **characterized in that** R₁ and R₂ are both methyl;
and/or R₃, R₄ and R₅ are all deuterium; and/or R is methyl.

3. The method according to claim 1 or 2, **characterized in that** in step (1), the reaction temperature is in the range of 40to 120 °C; and/or
in step (2), the reaction temperature is in the range of -10 to 60 °C; and/or
in step (3), the reaction temperature is in the range of 40 to 90 °C; and/or
in step (4), the reaction temperature is in the range of -10 to 70 °C; and/or in step (5), the reaction temperature is in the range of -10 to 40 °C.

4. The method according to claim 1 or 2, **characterized in that** in the cyclization of step (3), solvents are dimethyl sulfoxide or the mixture of dimethyl sulfoxide and isopropyl acetate, in which the volume ratio of dimethyl sulfoxide and isopropyl acetate is in the range of 50:1 to 1:10.

5. The method according to claim 4, **characterized in that** the volume ratio of dimethyl sulfoxide and isopropyl acetate is 1:2.

6. The method according to claim 1 or 2, **characterized in that** in step (4), said reaction is carried out in the presence of a base, and the base is selected from the alkali metal hydroxides.

7. The method according to claim 6, **characterized in that** said alkali hydroxides are selected from the group consisting of LiOH, KOH and NaOH, preferably LiOH.

8. The method according to claim 1 or 2, **characterized in that** in step (5), said amide condensation reaction is carried out in the presence of a condensing agent, and the condensing agent is selected from the group consisting of isopropyl chlorocarbonate, N,N'-carbonyldiimidazole and 2-(7-oxybenzotriazole)-N,N,N',N'-tetramethylurea hexafluorophosphate (HATU).

9. The method according to claim 1 or 2, **characterized in that** in step (1), said substitution reaction is carried out in alkaline environment, using Cu, CuI, and N,N-dimethylglycine as catalyst.

10. The method according to claim 1 or 2, **characterized in that** in step (2), said methyl esterification reagent for the carboxyl group is methyl iodide.

## Patentansprüche

1. Verfahren zur Herstellung von deuterierten Imidazoldiketonverbindungen, **dadurch gekennzeichnet, dass** es die folgenden Schritte enthält: 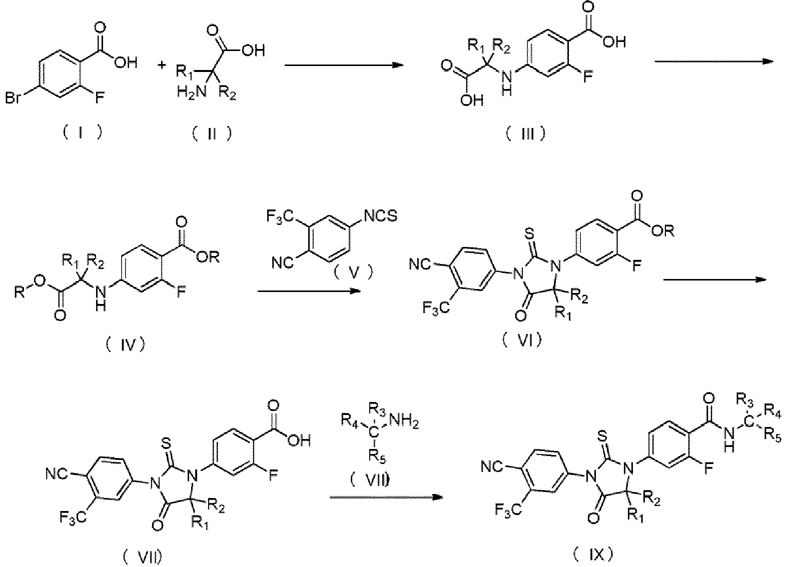
wobei R₁ und R₂ unabhängig aus C₁-C₄-Alkylgruppen ausgewählt sind;
R₃, R₄ und R₅ aus Wasserstoff und Deuterium ausgewählt sind, wobei mindestens eines davon Deuterium ist;
(1) Verwenden einer Verbindung der Formel (I) und von Verbindungen der Formel (II) als Ausgangsstoff, wobei Verbindungen der Formel (III) durch eine Substitutionsreaktion erhalten werden;
(2) Verbindungen der Formel (IV) werden durch Veresterung der Carboxylgruppe in Verbindungen der Formel (III) hergestellt, wobei R aus C₁-C₆-Alkylgruppen ausgewählt ist;
(3) die Cyclisierung von Verbindungen der Formel (IV) und der Verbindung der Formel (V) liefert Verbindungen der Formel (VI);
(4) Verbindungen der Formel (VI) werden entestert, was Verbindungen der Formel (VII) ergibt;
(5) Verbindungen der Formel (VII) und Verbindungen der Formel (VIII) werden umgesetzt, wobei die deuterierten Imidazoldiketonverbindungen der Formel (IX) durch eine Kondensationsreaktion unter Ausbildung einer Amidbindung erhalten werden.

2. Verfahren nach Anspruch 1, **dadurch gekennzeichnet, dass** R₁ und R₂ beide Methyl sind und/oder R₃, R₄ und R₅ alle Deuterium sind und/oder R Methyl ist.

3. Verfahren nach Anspruch 1 oder 2, **dadurch gekennzeichnet, dass** in Schritt (1) die Reaktionstemperatur im Bereich von 40 bis 120 °C liegt und/oder
in Schritt (2) die Reaktionstemperatur im Bereich von -10 bis 60 °C liegt und/oder
in Schritt (3) die Reaktionstemperatur im Bereich von 40 bis 90 °C liegt und/oder
in Schritt (4) die Reaktionstemperatur im Bereich von -10 bis 70 °C liegt und/oder
in Schritt (5) die Reaktionstemperatur im Bereich von -10 bis 40 °C liegt.

4. Verfahren nach Anspruch 1 oder 2, **dadurch gekennzeichnet, dass** bei der Cyclisierung von Schritt (3) als Lösungsmittel Dimethylsulfoxid oder die Mischung von Dimethylsulfoxid und Essigsäureisopropylester, in der das Volumenverhältnis von Dimethylsulfoxid zu Essigsäureisopropylester im Bereich von 50:1 bis 1:10 liegt, verwendet werden.

5. Verfahren nach Anspruch 4, **dadurch gekennzeichnet, dass** das Volumenverhältnis von Dimethylsulfoxid zu Essigsäureisopropylester 1:2 beträgt.

6. Verfahren nach Anspruch 1 oder 2, **dadurch gekennzeichnet, dass** in Schritt (4) die Umsetzung in Gegenwart einer Base durchgeführt wird und die Base aus den Alkalimetallhydroxiden ausgewählt wird.

7. Verfahren nach Anspruch 6, **dadurch gekennzeichnet, dass** die Alkalihydroxide aus der Gruppe bestehend aus LiOH, KOH und NaOH, vorzugsweise LiOH, ausgewählt werden.

8. Verfahren nach Anspruch 1 oder 2, **dadurch gekennzeichnet, dass** in Schritt (5) die Amid-Kondensationsreaktion in Gegenwart eines Kondensationsmittels durchgeführt wird und das Kondensationsmittel aus der Gruppe bestehend aus Chlorkohlensäureisopropylester, N,N'-Carbonyldiimidazol und 2-(7-Oxybenzotriazol)-N,N,N',N'-tetramethylharnstoff-hexafluorophosphat (HATU) ausgewählt wird.

9. Verfahren nach Anspruch 1 oder 2, **dadurch gekennzeichnet, dass** in Schritt (1) die Substitutionsreaktion in alkalischer Umgebung unter Verwendung von Cu, CuI und N,N-Dimethylglycin als Katalysator durchgeführt wird.

10. Verfahren nach Anspruch 1 oder 2, **dadurch gekennzeichnet, dass** es sich in Schritt (2) bei dem Methylveresterungsreagenz für die Carboxylgruppe um Methyliodid handelt.

## Revendications

1. Procédé pour la préparation de composés de type imidazole dicétone deutérés, **caractérisé en ce qu'**il comprend les étapes suivantes : 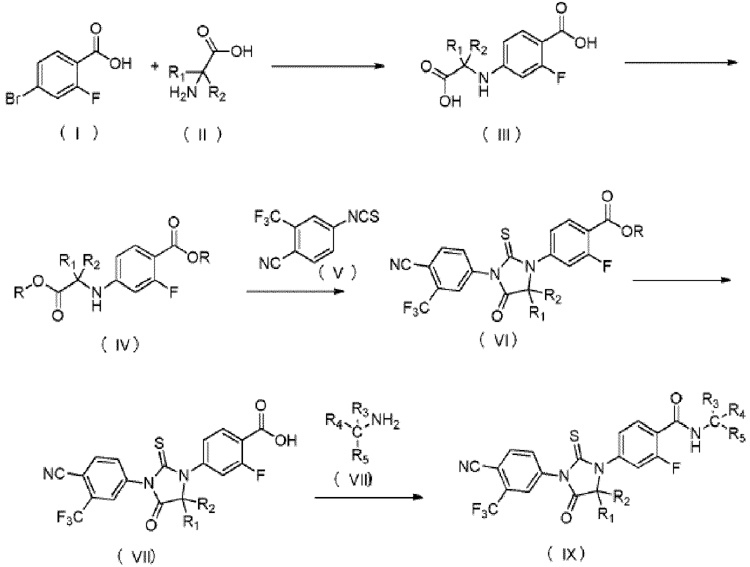
R₁ et R₂ étant indépendamment choisis parmi des C₁₋₄ alkyles ;
R₃, R₄ et R₅ étant choisis parmi hydrogène et deutérium, dans lesquels au moins l'un d'eux est deutérium ;
(1) utilisation d'un composé de formule (I) et de composés de formule (II) en tant que produit de départ, des composés de formule (III) étant obtenus par une réaction de substitution ;
(2) des composés de formule (IV) étant préparés par estérification du groupe carboxyle dans des composés de formule (III), R étant choisi parmi des C₁₋₆ alkyles ;
(3) une cyclisation de composés de formule (IV) et d'un composé de formule (V) fournissant des composés de formule (VI) ;
(4) des composés de formule (VI) étant désestérifiés pour produire des composés de formule (VII) ;
(5) des composés de formule (VII) et des composés de formule (VIII) étant mis à réagir, les composés de type imidazole dicétone deutérés de formule (IX) étant obtenus par une réaction de condensation formant une liaison amide.

2. Procédé selon la revendication 1, **caractérisé en ce que** R₁ et R₂ sont tous deux méthyle ; et/ou R₃, R₄ et R₅ sont tous deutérium ; et/ou R est méthyle.

3. Procédé selon la revendication 1 ou 2, **caractérisé en ce que** dans l'étape (1), la température de réaction est dans la plage de 40 à 120 °C ; et/ou
dans l'étape (2), la température de réaction est dans la plage de -10 à 60 °C ; et/ou
dans l'étape (3), la température de réaction est dans la plage de 40 à 90 °C ; et/ou
dans l'étape (4), la température de réaction est dans la plage de -10 à 70 °C ; et/ou dans l'étape (5), la température de réaction est dans la plage de -10 à 40 °C.

4. Procédé selon la revendication 1 ou 2, **caractérisé en ce que** dans l'étape de cyclisation (3), des solvants sont le diméthylsulfoxyde ou le mélange de diméthylsulfoxyde et d'acétate d'isopropyle, dans lequel le rapport volumique de diméthylsulfoxyde et d'acétate d'isopropyle est dans la plage de 50 : 1 à 1 : 10.

5. Procédé selon la revendication 4, **caractérisé en ce que** le rapport volumique de diméthylsulfoxyde et d'acétate d'isopropyle est de 1 : 2.

6. Procédé selon la revendication 1 ou 2, **caractérisé en ce que** dans l'étape (4), ladite réaction est mise en œuvre en la présence d'une base, et la base est choisie parmi les hydroxydes de métaux alcalins.

7. Procédé selon la revendication 6, **caractérisé en ce que** lesdits hydroxydes alcalins sont choisis dans le groupe constitué par LiOH, KOH et NaOH, préférablement LiOH.

8. Procédé selon la revendication 1 ou 2, **caractérisé en ce que** dans l'étape (5), ladite réaction de condensation d'amide est mise en œuvre en la présence d'un agent de condensation, et l'agent de condensation est choisi dans le groupe constitué par le chlorocarbonate d'isopropyle, le N,N'-carbonyldiimidazole et l'hexafluorophosphate de 2-(7-oxybenzotriazole)-N,N,N',N'-tétraméthylurée (HATU).

9. Procédé selon la revendication 1 ou 2, **caractérisé en ce que** dans l'étape (1), ladite réaction de substitution est mise en œuvre dans un environnement alcalin, en utilisant Cu, CuI et la N,N-diméthylglycine en tant que catalyseur.

10. Procédé selon la revendication 1 ou 2, **caractérisé en ce que** dans l'étape (2), ledit réactif d'estérification méthylique pour le groupe carboxyle est l'iodure de méthyle.
